# EUROPEAN PATENT SPECIFICATION

(11) **EP 3 721 896 B1**
(45) Date of publication and mention of the grant of the patent: **29.11.2023**
(21) Application number: 19167967.9
(22) Date of filing: 08.04.2019
(51) Int. Cl.: A61K 38/18, A61P 25/28, G01N 33/68

(54) **EPIDERMAL GROWTH FACTOR (EGF) AND VARIANTS THEREOF FOR TREATING OR PREVENTING INFLAMMATORY DEMYELINATING DISORDERS**
EPIDERMALER WACHSTUMSFAKTOR (EGF) UND VARIANTEN DAVON ZUR BEHANDLUNG ODER VORBEUGUNG VON INFLAMMATORISCHEN DEMYELINISIERENDEN ERKRANKUNGEN
FACTEUR DE CROISSANCE ÉPIDERMIQUE (EGF) ET SES VARIANTES POUR LE TRAITEMENT OU LA PRÉVENTION DE TROUBLES INFLAMMATOIRES DE DEMYELINISATION

(43) Date of publication of application: 14.10.2020
(73) Proprietor: Scalabrino, Giuseppe, 20122 Milano (IT); Nicoletti, Ferdinando, 95021 Aci Castello, Catania (IT); Lang, Alois Bernhard, 3033 Wohlen b. Bern (CH); Curtin, Francois, 1292 Chambesy (CH)
(72) Inventor: Scalabrino, Giuseppe, 20122 Milano (IT); Nicoletti, Ferdinando, 95021 Aci Castello, Catania (IT); Lang, Alois Bernhard, 3033 Wohlen b. Bern (CH); Curtin, Francois, 1292 Chambesy (CH)
(74) Representative: Vossius & Partner Patentanwälte Rechtsanwälte mbB

(56) References cited:
- EP-A1- 1 889 852
- EP-A2- 1 870 106
- US-A1- 2002 004 039
- DEL BARCO DIANA GARCÍA ET AL: "Coadministration of epidermal growth factor and growth hormone releasing peptide-6 improves clinical recovery in experimental autoimmune encephalitis.", RESTORATIVE NEUROLOGY AND NEUROSCIENCE 2011, vol. 29, no. 4, 2011, pages 243-252, XP55619892, ISSN: 1878-3627
- GOVINDAIAH VINUKONDA ET AL: "Epidermal growth factor preserves myelin and promotes astrogliosis after intraventricular hemorrhage : Epidermal Growth Factor and IVH", GLIA, vol. 64, no. 11, 29 July 2016 (2016-07-29) , pages 1987-2004, XP055620010, ISSN: 0894-1491, DOI: 10.1002/glia.23037
- ALMAZAN G ET AL: "Epidermal growth factor and bovine growth hormone stimulate differentiation and myelination of brain cell aggregates in culture", DEVELOPMENTAL BRAIN RESEARCH, ELSEVIER SCIENCE BV, AMSTERDAM, NL, vol. 21, no. 2, 1 August 1985 (1985-08-01) , pages 257-264, XP025413654, ISSN: 0165-3806, DOI: 10.1016/0165-3806(85)90214-7 [retrieved on 1985-08-01]
- KNAPP P E ET AL: "Epidermal growth factor promotes oligodendrocyte process formation and regrowth after injury", EXPERIMENTAL CELL RESEARCH, ELSEVIER, AMSTERDAM, NL, vol. 296, no. 2, 18 March 2004 (2004-03-18), pages 135-144, XP002395583, ISSN: 0014-4827, DOI: 10.1016/J.YEXCR.2004.02.007
- MARTA TEJERA-ALHAMBRA ET AL: "Plasma Biomarkers Discriminate Clinical Forms of Multiple Sclerosis", PLOS ONE, vol. 10, no. 6, 3 June 2015 (2015-06-03), page e0128952, XP055619754, DOI: 10.1371/journal.pone.0128952
- SCALABRINO GIUSEPPE ET AL: "Relationships between cobalamin, epidermal growth factor, and normal prions in the myelin maintenance of central nervous system", INTERNATIONAL JOURNAL OF BIOCHEMISTRY AND CELL BIOLOGY, PERGAMON, GB, vol. 55, 17 September 2014 (2014-09-17), pages 232-241, XP029020040, ISSN: 1357-2725, DOI: 10.1016/J.BIOCEL.2014.09.011
- NICOLETTI FERDINANDO ET AL: "Prevention of clinical and histological signs of MOG-induced experimental allergic encephalomyelitis by prolonged treatment with recombinant human EGF", JOURNAL OF NEUROIMMUNOLOGY, vol. 332, 8 May 2019 (2019-05-08), pages 224-232, XP085694222, ISSN: 0165-5728, DOI: 10.1016/J.JNEUROIM.2019.05.006

## Description

The present invention is defined by the appended claims. Generally described herein is a (poly)peptide having Epidermal Growth Factor (EGF)-activity or a nucleic acid encoding said (poly)peptide for use in treating or preventing a demyelinating and/or neurodegenerating disorder, wherein the (poly)peptide (a) comprises or consists of the amino acid sequence of SEQ ID NO: 1; and/or (b) comprises or consists of an amino acid sequence, which is at least 66% identical to the amino acid sequence of SEQ ID NO: 1; and/or (c) comprises or consists of a fragment of (a) or (b); and wherein the (poly)peptide or nucleic acid is the only pharmaceutically active agent to be administered.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Demyelinating disorders encompass a broad range of pathologies in which myelin, the lipid substance that forms an insulating sheath around nerve fibers (axons), is damaged, lost, or exhibits impaired growth or development. As myelination is critical for the rapid conduction of nerve impulses throughout the nervous system, demyelination causes a breakdown in conduction and results in various functional impairments. In absence of a functional myelin sheath, nerve impulses cannot be properly transmitted - they either travel too slowly or not at all. Axons that have been demyelinated (e.g., upon chronic demyelination) tend to atrophy and may eventually degenerate, leading to nerve cell death (Love S. J Clin Pathol. (2006);59(11):1151-9). There are also neurodegenerating disorders, which are commonly hold distinct from classical demyelinating disorders, in which axonal degradation occurs first and degradation of myelin is secondary.

Subjects suffering from demyelinating and/or neurodegenerating disorders may experience muscle weakness, loss of sensation, impaired vision, impaired cognition, ataxia, and paralysis, among other symptoms. Signs and symptoms can range from relatively mild to profound, producing severe reductions in quality of life and possibly death. Current standards for treating certain demyelinating and/or neurodegenerating disorders, such as multiple sclerosis (MS), are mostly based on either immune suppression or immune modulation, primarily affecting the rate of disease progression, and typically have no direct effects on nerve repair (e.g., remyelination).

Thus, there is a need for new therapeutic approaches for the treatment of demyelinating and/or neurodegenerating disorders, in particular for preventing demyelination or promoting remyelination. The present invention addresses this need and provides related advantages as well.

The present invention is defined by the claims. The present invention relates in a first aspect to a (poly)peptide having Epidermal Growth Factor (EGF)-activity or a nucleic acid encoding said (poly)peptide for use in treating or preventing an inflammatory demyelinating disorder, wherein the (poly)peptide (a) comprises or consists of the amino acid sequence of SEQ ID NO: 1; and/or (b) comprises or consists of an amino acid sequence, which is at least 66% identical to the amino acid sequence of SEQ ID NO: 1; and/or (c) comprises or consists of a fragment of (a) or (b); and wherein the (poly)peptide or nucleic acid is the only pharmaceutically active agent to be administered.

The term "(poly)peptide" in accordance with the present invention describes a group of molecules which comprises the group of peptides, consisting of up to 30 amino acids, as well as the group of polypeptides, also referred to as proteins, consisting of more than 30 amino acids. The term "fragment of the (poly)peptide" in accordance with the present invention refers to a portion of the (poly)peptide comprising at least the amino acid residues necessary to maintain the biological activity of the (poly)peptide. (Poly)peptides may further form dimers, trimers and higher oligomers, *i.e.,* consisting of more than one (poly)peptide molecule. (Poly)peptide molecules forming such dimers, trimers etc. may be identical or non-identical. The corresponding higher order structures are, consequently, termed homo- or heterodimers, homo- or heterotrimers etc. Homo- or heterodimers etc. also fall under the definition of the term "(poly)peptide". The terms "(poly)peptide" and "protein", used interchangeably herein, also refer to chemically modified (poly)peptides.

Chemical modifications encompassed herein include any covalent modification (*i.e*., including natural modifications, e.g., post-translational modifications) that may alter, preferably improve, the pharmacological and/or pharmacodynamic properties of the (poly)peptide, *i.e*., the modification may be effected, e.g., by glycosylation, acetylation, phosphorylation and the like. (Poly)peptides which are albuminated (covalent attachment of albumin) or PEGylated (*i*.*e*., covalent attachment of polyethylene glycol polymer chains) are also encompassed by the present invention. The skilled person is aware of suitable methods to introduce one or more of these modifications.

The term "altered pharmacological and/or pharmacodynamic properties" as used herein refers, for example, but not limited to, altered oral bioavailability, pharmacokinetics (e.g., increased or decreased affinity to EGFR), distribution, targeting capability, serum half-life, solubility, stability (e.g., thermal stability, proteolytic, stability etc.), tissue penetration, and/or blood-brain barrier penetration, as compared to the unmodified (poly)peptide.

Thus, the (poly)peptides provided herein can contain one or more modifications that introduces one or more glycosylation sites compared to the unmodified (poly)peptide. In some examples, the glycosylation sites are selected from among, N-, O- and S-glycosylation sites. In one example, one or more N-glycosylation sites are introduced compared to the unmodified (poly)peptide.

Furthermore, peptidomimetics of such (poly)peptides, where amino acid(s) and/or peptide bond(s) have been replaced by functional analogues, are also encompassed herein. Such functional analogues include all known amino acids other than the 20 genetically-encoded amino acids, such as selenocysteine and pyrrolysine.

The term "nucleic acid" as used herein interchangeably with the terms "nucleic acid molecule" or "polynucleotide", includes DNA, such as cDNA or genomic DNA, and RNA. It is understood that the term "RNA" as used herein comprises all forms of RNA including mRNA.

EGF was discovered by Dr. Stanley Cohen more than half a century ago and it represents the prototypical member of a family of peptide growth factors that activate the EGF receptors. EGF represents the prototype of the group I EGF family that also includes transforming growth factor (TGF)-α, heparin-binding EGF (HB-EGF), amphiregulin, betacellulin, epiregulin and epigen (Zeng F & Harris RC, Semin Cell Dev Biol. (2014);28:2-11).

EGF binds to a specific receptor, Epidermal Growth Factor Receptor (EGFR), also known as ErbB1/HER1, that belongs to the epidermal growth factor (EGF) family of receptor tyrosine kinases (RTKs) also called ErbB or HER family of receptors, which also includes ErbB2/HER2/Neu, ErbB3/HER3, and ErbB4/HER4 (Wee P, Wang Z, Cancers (Basel) (2017);9(5):52; Wieduwilt MJ, Moasser MM., Cell Mol Life Sci. (2008);65(10):1566-84).

EGFR is a single-chain transmembrane glycoprotein, which comprises an extracellular ligand-binding ectodomain, a transmembrane domain, a short juxtamembrane section, a tyrosine kinase domain and a tyrosine-containing C-terminal tail. Binding of soluble EGF to the ectodomain of its receptor (EGFR) promotes homodimer formation between EGFR receptors. EGFR may, upon EGF binding, also pair with another member of the ErbB receptor family to form a heterodimer. Receptor dimerization is essential for activation of the intracellular tyrosine kinase domain and phosphorylation of the C-terminal tail. Phosphotyrosine residues then activate, either directly or through adaptor proteins, downstream components of signaling pathways including Ras/MAPK, PLCy1/PKC, PI(3)kinase/Akt, and STAT pathways (Wieduwilt MJ, Moasser MM., Cell Mol Life Sci. (2008);65(10):1566-84).

In accordance with the present invention, the specified requirement that the (poly)peptide has "Epidermal growth factor (EGF) activity" is to be understood that the polypeptide must maintain its capacity to bind EGFR and to activate its signalling. Multiple tests are known in the art that can be employed for assessing the presence or absence of EGF activity of a candidate (poly)peptide, for example, by assessing its capacity to induce proliferation of mouse fibroblast cell lines BALB/3T3, i.e., by assessing the growth response of these cells in response to various amounts of candidate (poly)peptide (Brown KD & Holley RW, J Cell Physiol. (1979);100(1):139-46).

Alternatively, suitable (poly)peptides having "Epidermal growth factor (EGF) activity" can be identified by assessing their capacity to prevent demyelination and/or to induce amelioration of the clinical course of a demyelinating and/or neurodegenerating disease (e.g. by using animal models for demyelinating and/or neurodegenerating disorders, such as the MOG-induced EAE mouse model as described herein, and evaluating the clinical course of the disease using the evaluation criteria as described herein (see Examples 1 and 2)). Similarly, suitable (poly)peptides having "EGF activity" can also be identified using the herein described animal model and detecting or assessing the degree of inflammation or demyelination using histological (histopathological) analyses (see Examples 1 and 2).

It is further understood that the (poly)peptide according to the first aspect of the invention is preferably soluble, i.e., not membrane anchored (e.g., by having an integral transmembrane domain).

Structurally, EGF is a 6.2-kDa single-chain polypeptide of 53 amino acids that has six conserved cysteines which form three intramolecular disulfide bonds, with the following pairing: C₆-C₂₀, C₁₄-C₃₁, and C₃₃-C₄₂ (C, cysteine; numbering according to the amino acid sequence of human EGF). EGF is initially synthesized as a transmembrane glycoprotein precursor (prepro-EGF), and then proteolytically processed to mature EGF protein.

The (poly)peptide having Epidermal growth factor (EGF)-activity is, or is derived from, SEQ ID NO: 1 (NSDSECPLSHDGYCLHDGVCMYIEALDKYACNCVVGYIGERCQYRDLKWWELR), which corresponds to the amino acid sequence (amino acid residues 1 to 53) of mature human EGF, corresponding to the amino acid residues 971 to 1023 of the prepro-precursor of human EGF (numbering of prepro-EGF according to UniProt Database entry: P01133-1; February 15, 2019 (see https://www.uniprot.org/uniprot/P01133)).

The term "percent (%) sequence identity" in accordance with the present invention describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 70%, 75%, 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

In accordance with the present invention, a "demyelinating disorder" is a condition in which the myelin sheath which surrounds neurons in nervous tissue is lost or damaged, leading to axonal degeneration and impaired signal transduction in the affected nerves. Examples of demyelinating diseases include multiple sclerosis (MS), transverse myelitis, Guillain-Barré syndrome (GBS), optic neuritis, neuromyelitis optica, acute disseminated encephalomyelitis, adrenoleukodystrophy, adrenomyeloneuropathy, idiopathic inflammatory demyelinating disease, central pontine myelinolysis, and progressive multifocal leukoencephalopathy.

By "neurodegenerative disorder" is meant any disease or disorder caused by or associated with the deterioration of cells or tissues of the nervous system. Exemplary neurodegenerative disorders are polyglutamine expansion disorders (e.g., HD, dentatorubropallidoluysian atrophy, Kennedy's disease (also referred to as spinobulbar muscular atrophy), and spinocerebellar ataxia (e.g., type 1, type 2, type 3 (also referred to as Machado-Joseph disease), type 6, type 7, and type 17)), other trinucleotide repeat expansion disorders (e.g., fragile X syndrome, fragile XE mental retardation, Friedreich's ataxia, myotonic dystrophy, spinocerebellar ataxia type 8, and spinocerebellar ataxia type 12), Alexander disease, Alper's disease, Alzheimer disease (AD), amyotrophic lateral sclerosis, ataxia telangiectasia, Batten disease (also referred to as Spielmeyer-Vogt-Sjögren-Batten disease), Canavan disease, Cockayne syndrome, corticobasal degeneration, Creutzfeldt-Jakob disease, ischemia stroke, Krabbe disease, Lewy body dementia, multiple sclerosis (MS), multiple system atrophy, Parkinson's disease, Pelizaeus-Merzbacher disease, Pick's disease, primary lateral sclerosis, Refsum's disease, Sandhoff disease, Schilder's disease, spinal cord injury, spinal muscular atrophy, Steele-Richardson-Olszewski disease, and Tabes dorsalis.

The term "disorder", as used herein, refers to any condition or disease or pathology (in particular, neuropathy and/or polyneuropathy) that would benefit from, or could be prevented by, the treatment according to the first aspect of the invention. Multiple sclerosis (MS) and many neurodegenerative disorders are diagnosed by clinical evaluation, specific neurologic tests allowing determination of scores on clinical scales plus magnetic resonance imaging (MRI) of the brain. For peripheral neuropathies, clinical signs and scores on clinical scales completed by laboratory assessment such as nerve conductivity are needed.

Human EGF shares 70%, 70%, 83%, and 66% amino acid sequence identity with mouse, rat, porcine and horse EGF, respectively. As shown in the examples of the present invention, recombinant human EGF (Sigma Aldrich, Milan, Italy) having the amino acid sequence of SEQ ID NO: 1 proved to be effective in the experimental murine animal model (see Examples 1 and 2). It is hence expected that (poly)peptides having, or being derived from, the amino acid sequence of murine EGF, or of any other (poly)peptide having at least 66% identity to human EGF (SEQ ID NO: 1), will also possess the described beneficial EGF-activity, when administered to a human or other mammalian subject.

Thus, the (poly)peptide according to item (b) of the first aspect of the invention comprises or consists of an amino acid sequence, which is at least 66% identical to the amino acid sequence of SEQ ID NO: 1. The (poly)peptide according to item (b) of the first aspect of the present invention may comprise or consist of an amino acid sequence which is at least 70% identical, more preferably at least 80% identical, even more preferably at least 90% identical, and most preferred at least 95% identical to the amino acid sequence of SEQ ID NO: 1. However, also envisaged are with increasing preference sequence identities of at least 97.5%, at least 98.5%, at least 99%, at least 99.5%, at least 99.8%, and 100 %.

The fragment according to item (c) of the first aspect of the invention consists, with increasing preference, of at least 17, at least 20, at least 33, or at least 35, and even more preferred of least 40, and most preferred of at least 45 amino acids. It is self-explanatory that the fragments encompassed by the present invention also have EGF-activity in accordance with the definition of the term "EGF-activity" provided herein above. For example, in WO1992/003476, various peptides and peptide analogs having biological activity comparable to that of native human EGF and having the amino acid sequences of, or similar to that of, native human EGF amino acid residues 32 to 48 (corresponding to SEQ ID NO: 2 (NCVVGYIGERCQYRDLK)) are described.

It is known that EGF and the other members of the group I EGF family (including TGF-α, HB-EGF, amphiregulin, betacellulin, epiregulin and epigen) each comprise six conserved cysteine residues, one conserved glycine and one conserved arginine in the following pattern: CX₇CX₃₋₅CX₁₀₋₁₂CXCX₅GXRC (C, cysteine; G, glycine; R, arginine; X, other amino acids), wherein the cysteines form three intramolecular disulfide bonds with the following pairing C₁-C₃, C₂-C₄ and C₅-C₆ (numbered according to their order in the sequence) (Zeng F & Harris RC.; Semin Cell Dev Biol. (2014);28:2-11).

Thus, in certain cases, (i) the amino acid sequence which is at least 66% identical to the amino acid sequence of SEQ ID NO: 1, and/or (ii) the fragment consisting of at least 35 amino acids, may comprise six cysteines, one glycine and one arginine in the following pattern: CX₇CX₃₋₅CX₁₀₋₁₂CXCX₅GXRC (C, cysteine; G, glycine; R, arginine; X, any other amino acid than cysteine), wherein the cysteines are involved in three intramolecular disulfide bonds with the following pairing: C₁-C₃, C₂-C₄ and C₅-C₆ (numbered according to their order in the sequence), as present in wild-type EGF (SEQ ID NO: 1).

The amino acid sequence which is at least 66% identical to the amino acid sequence of SEQ ID NO: 1, and/or the fragment consisting of at least 35 amino acids, may comprise six cysteines, one glycine and one arginine in the following pattern: CX₇CX₅CX₁₀CXCX₅GXRC (C, cysteine; G, glycine; R, arginine; X, any other amino acid than cysteine), wherein the cysteines are involved in three intramolecular disulfide bonds with the following pairing: C₁-C₃, C₂-C₄ and C₅-C₆ (numbered according to their order in the sequence), as present in wild-type EGF (SEQ ID NO: 1).

By the specification that the (poly)peptide or nucleic acid "is the only pharmaceutically active agent to be administered", in accordance with the first aspect of the present invention, it is in particular excluded that any one or more of growth hormone releasing peptide-6 (GHRP-6), tumor necrosis factor (TNF)-α, cobalamin and/or leukaemia inhibitory factor (LIF) is/are administered together with the polypeptide according to the present invention. This exclusion also applies to nucleic acids encoding one or more of GHRP-6, LlF and/or TNF-α.

EGFR is a tyrosine kinase that is commonly upregulated in cancers such as in non-small-cell lung cancer, metastatic colorectal cancer, glioblastoma, head and neck cancer, pancreatic cancer, and breast cancer. Due to its capacity to induce cell proliferation, while inhibiting apoptosis, the EGF/EGFR signaling system has been classically identified as pro-oncogenic. This has led to the development of specific inhibitors (such as monoclonal antibodies (mAbs)) of either EGF, or its receptors, that are currently used for the treatment of certain forms of cancers such as breast cancer and colorectal cancer.

Due to its pleiotropic effects, EGF has attracted attention as to the possibility that a dysregulated expression of this peptide and/or its receptor may also be implicated in other (non-neoplastic) conditions, including immunoinflammatory and autoimmune diseases. Much interest has been devoted to studying the role of EGF in the development and progression of multiple sclerosis (MS) that is a prototypical neuroinflammatory disease characterized by demyelination and often followed by neurodegeneration.

For example, EGF was suggested to be one of several other factors contributing to myelin maintenance *(*Scalabrino G, et al., (2014), Int J Biochem Cell Biol.;55:232-41)*.* Moreover, it was reported that EGF stimulates oligodendrocytes (ODC) process formation (Chandran S, et al., Glia. (1998);24(4):382-9*;* Pfeiffer SE, et al., Trends Cell Biol. (1993);3(6):191-7), especially after a CNS injury (Knapp PE, et al., Exp Cell Res. (2004) 10;296(2):135-44*)* and that EGF when administrated immediately after neonatal mouse brain injury enhances ODC proliferation from progenitor cells *(*Scafidi J, et al., Nature (2014);506(7487):230-4*).* In addition, it was reported that EGF treatment increases the ODC number derived from subventricular zone type B-cells after a lysolecithin-induced demyelination of mouse corpus callosum (Gonzalez-Perez O, et al., Stem Cells. (2009);27(8):2032-43). In another study, genetic polymorphisms in the EGF and Tyk2 genes were identified as potential determinants of CNS repair (Bieber AJ, et al., Proc Natl Acad Sci U S A. (2010);107(2):792-7*).* Moreover, EGF was reported to act as mediator of the myelinotrophic action of cobalamin (Scalabrino G, et al., Epidermal growth factor as a local mediator of the neurotrophic action of vitamin B(12) (cobalamin) in the rat central nervous system, FASEB J. (1999);13(14):2083-90); and a different study reported that repeated intracerebroventricular (ICV) microinjections of specific anti-EGF antibodies induce myelin lesions of the white matter of the spinal cord in normal rats (Scalabrino G, et al., J Neuropathol Exp Neurol. (2000);59(9):808-14).

Several other studies revealed that EGF together with a variety of other factors is aberrantly expressed in demyelinating disease conditions. For example, one study reported that mRNA levels of EGF and various other growth factors are increased in the CNS of mice during remyelination after cuprizone-induced demyelination (Gudi V, et al. PLoS One. 2011;6(7):e22623)*.* A further study reported decreased levels of EGF and of tumor necrosis factor (TNF)-α and increased levels of cobalamin (Cbl) in cerebrospinal fluid (CSF) (but not in serum) of MS patients (Scalabrino G, et al.; Brain Res. 2010 May 28;1333:64-71). A subsequent study reported decreased levels of EGF, Cbl and normal cellular prions (PrP(C)s) in spinal cord (SC) samples from MS patients (Scalabrino G., et al., Neuroscience (2015), 298:293-301). A more recent study reported that blood levels of EGF (and of the β-chemokine MIP-1β/CCL4) are significantly decreased in patients with secondary progressive (SP) MS with respect to patients with relapsing-remitting (RR) MS, and suggested EGF together with MIP-1β/CCL4 as protective factors for developing progressive clinical forms of MS (Tejera-Alhambra M, et al., PLoS One (2015);10(6):e0128952).

In conclusion of these earlier reports, EGF was generally considered as being only one factor within an intricate network of multiple other factors collectively implicated and orchestrating the underlying processes of myelination and dysregulated in demyelinating diseases. However, none of these studies has considered the possibility, or even proven, that EGF may be effective in preventing demyelination and promoting remyelination already when administered alone.

In fact, several preclinical studies questioned or contradicted a potential neuroprotective role of EGF. Most strikingly, in one study, the course of MOG-induced EAE was ameliorated upon treatment with a specific anti-EGF antibody (Ab) (Amir-Levy Y, et al., Mult Scler Int. (2014);2014:926134). In another study, exogenously-administered EGF was only capable of ameliorating the course of experimental autoimmune encephalomyelitis (EAE), in Lewis rat when administered in a combined treatment with growth hormone-releasing peptide-6 (GHRP-6) (del Barco DG, et al., J. Restor Neurol Neurosci. (2011);29(4):243-52; and EP1870106A2). This dependency was explained by the applicants of EP1870106A2 by a synergic effect between EGF and GHRP-6 (cf. paragraph [0019] in EP1870106A2). Notably, upon administration of either EGF or GHRP-6 alone, no effect was observed as compared to treatment with placebo (cf. paragraph [0029] and table 1 of EP1870106A2). It should, however, be noticed that the validity of Lewis rat EAE as a preclinical model of human MS has been questioned due to the lack of demyelination that is observed in the human disease counterpart and the monophasic and self-remitting course of the disease that contrasts with the characteristic chronic progressivity of MS in humans (Shin T, et al., Mechanism of experimental autoimmune encephalomyelitis in Lewis rats: recent insights from macrophages. Anat Cell Biol.(2012);45(3):141-8). In contrast to these previous reports (del Barco DG, et al. and EP1870106A2), the present inventors surprisingly and unexpectedly found that EGF provides for these neuroprotective effects also (and already) when administered alone. The absence of a neuroprotective effect when EGF was administered alone (as reported by del Barco DG, et al. and EP1870106A2) may be explained, without being bound by any theory, by a (at least partial) neutralization of administered recombinant human EGF by anti-drug-antibodies (ADA) elicited in the rodent animal model in response to the foreign human recombinant protein.

The present inventors set out to evaluate the feasibility of treatment with exogenous EGF to counteract demyelinating lesions and eventually promote remyelination. A dose-finding analysis of recombinant human EGF in a well-known model of mouse EAE induced by immunization with myelin oligodendrocyte glycoprotein (MOG) was conducted. MOG-induced EAE is characterized by progressive course of the diseases that is well known to be histologically associated with inflammatory infiltrates of the spinal cords and brain along with marked areas of demyelination (Mangano K, et al., J Cell Physiol. (2014);229(12):1918-25*;* Eugster HP, et al., Eur J Immunol. (1999);29(2):626-32). The present findings demonstrate for the first time that *in vivo* treatment with EGF ameliorated clinical and histological signs of MOG-induced EAE. As apparent from the appended experimental data, EGF when administered alone was capable of reverting an actively ongoing auto-reactive demyelination process that has led to aggressive development of EAE and associated prototypical histological signs that include inflammatory infiltration of spinal cords and demyelination.

Taken together the data herein disclosed provide clear-cut *in vivo* proof of concept for a beneficial role of exogenously administered EGF in an established animal model of demyelinating disease. The combined finding of improved clinical development of the disease with absent demyelination suggests that treatment with EGF, administrated as sole pharmaceutically active agent, represents a novel approach for treatment of demyelinating and/or neurodegenerating disorders, such as multiple sclerosis, in particular, with the aim to prevent demyelination and/or to promote remyelination.

In a preferred embodiment of the first aspect of the invention, the fragment according to item (c) comprises or consists of SEQ ID NO: 2 (NCWGYIGERCQYRDLK), corresponding to amino acid residues 32 to 48 of native human EGF (SEQ ID NO: 1). This particular peptide of SEQ ID NO: 2 is described in WO1992/003476 to have biological activity comparable to that of native human EGF. The fragment may be cyclic (due to disulfide bridging of the two internal cysteines).

The (poly)peptide of the invention may be formulated for administration according to the invention as part of a pharmaceutical composition. It is to be understood that the pharmaceutical composition in the context of the present invention does not comprise any further pharmaceutically active agent besides the (poly)peptide of the present invention.

For parenteral administration, the pharmaceutical composition is formulated generally by mixing the (poly)peptide of the invention at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; serum albumin, gelatin; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The (poly)peptide of the invention may be administered along with a carrier or under conditions that enhance passage of the (poly)peptide across the blood brain barrier (BBB). For example, a blood brain barrier permeabilizer can be utilized. Blood brain barrier permeabilizers are known in the art and include, by way of example, bradykinin and the bradykinin agonists described in U.S. Pat. Nos. 5,686,416; 5,506,206 and 5,268,164 (such as NH₂-arginine-proline-hydroxyproxyproline-glycine-thienylalanine-serine-proline-4-Me-tyrosineψ(CH₂NH)-arginine-COOH). Alternatively, the molecules to be delivered can be conjugated to transport vectors including for example, the transferrin receptor antibodies (as described in U.S. Pat. Nos. 6,329,508; 6,015,555; 5,833,988 or 5,527,527), cationized albumin, insulin, insulin-like growth factors (IGF-I, IGF-II), angiotensin II, atrial and brain natriuretic peptide (ANP, BNP), interleukin I (IL-I), transferrin, cationized LDL, albumin or horseradish peroxidase coupled with polylysine, cationized albumin, cationized immunoglobulin, small basic oligopeptides (e.g., dynorphin analogue E-2078 or the ACTH analogue ebiratide), hexose moieties (e.g., glucose), monocarboxylic acids (e.g., lactic acid). The (poly)peptide can also be delivered as a fusion protein comprising the molecule and a ligand that is reactive with a brain capillary endothelial cell receptor, such as the transferrin receptor (see, e.g., U.S. Pat. No. 5,977,307). The above specified "carriers", "transport vectors" do not fall under the term "pharmaceutically active agent" as used herein, and, thus, are not excluded from being co-administered with the (poly)peptide in accordance with the first aspect of the invention.

The (poly)peptide according to the invention can be administered systemically (e.g., orally, rectally, parenterally (e.g., intravenously), intramuscularly, intraperitoneally, transdermally (e.g., by a patch), topically (as by powders, ointments, drops or transdermal patch), bucally, or as an oral or nasal spray, by inhalation, subcutaneously or the like), by administration into the central nervous system (e.g., into the brain (e.g., intracerebrally, intraventricularly or intracerebroventricular) or spinal cord or into the cerebrospinal fluid), or any combination thereof.

The (poly)peptide may be administered in one or more dose administrations, at least once daily, for one or more days (e.g., 1, 2, 3, 4, 5, 6, 7 days; 2, 3, 4 weeks; 2, 3, 4, 5, 6, 7, 8, 9, 10, 11, 12 months; 2 years, 3 years, 4 years, or longer). However, the skilled person understands that the duration of the administration, in accordance with the present invention, can optionally be sustained for the remaining lifetime of the subject. The (poly)peptide may be administered daily or less frequently, for example, every-other-day, semi-weekly, weekly, biweekly, semi-monthly, monthly, bimonthly, etc.

In a preferred embodiment of the first aspect of the invention, an effective amount of the (poly)peptide is to be administered to a subject in need thereof once every 36 hours to once every 60 hours, more preferably once every 44 hours to once every 52 hours, and most preferred once every about 48 hours. In connection with the present invention, it was found that the treatment of MOG-induced EAE mice (an animal model of demyelinating/neurodegenerating diseases, in particular MS) with EGF was particularly effective in ameliorating the clinical course of the disease and preventing the histological signs of demyelination when administered every-other-day (EOD), as detailed in Example 2.

In a preferred embodiment of the first aspect of the invention, the (poly)peptide is to be administered for a period of at least 20 days, preferably at least 30 days, more preferably at least 40 days. As apparent from the herein disclosed experimental data of the mouse EAE model, amelioration of clinical signs due to EGF treatment (EGF was administered from day 7 after immunization with MOG antigen) was observed already from the first day (day 9 after MOG-immunization) where clinical disease symptoms are usually observable in this EAE model (and as were observed in the vehicle-treated control group) until the end of the study (day 46 after MOG-immunization); see Example 2. The (poly)peptide of the invention can be administered to the subject at a suitable dose and/or a therapeutically effective amount. This will be further discussed herein below.

The dosage of the (poly)peptide of the invention required will somewhat vary from subject to subject, depending on the species, age, weight, and general condition of the subject, the particular active agent and pharmaceutical carrier used, the mode of administration and the like. For example, the dose of the (poly)peptide when administered parenterally may be in the range of about 1 µg (poly)peptide /kg to 10 mg (poly)peptide /kg of patient body weight (bd wt), although, as noted above, this will be subject to therapeutic discretion. More preferably, this dose is at least 0.01 mg (poly)peptide /kg/day, and most preferably for humans between about 0.01 and 5 mg (poly)peptide /kg/day. If given continuously, the pharmaceutical composition is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

The dosage of the (poly)peptide of the invention required will vary from subject to subject, depending on the species, age, weight, and general condition of the subject, the particular active agent used, its mode of administration and the like. For example, animal models for demyelinating/neurodegenerating disorders (e.g., the EAE mouse model, cuprizone induced demyelination model and lysolecithin model) can be used to monitor levels of response. Clinical signs can be monitored, and end point histopathological analyses of inflammation, demyelination and/or axonal disruption can be determined in subjects with or in animal models of demyelinating and/or neurodegenerating disorders as described in the herein disclosed examples. Histological tests can be used to detect, for example, myelin, OPCs or oligodendrocyte numbers, remyelination, cytokine levels, lymphocyte number. Cell sorting can be performed to identify lymphocyte numbers in tissue samples. Imaging (e.g., magnetic resonance imaging (MRI)) and functional measurements (survival or clinical symptoms) can be used to monitor a subject's response to therapy. Furthermore, resected human brain samples (e.g., samples derived as a means to treat an otherwise intractable disorders) can be dissociated and cultured as neurospheres to study the effect of doses of the (poly)peptide of the invention. Electrophysiology studies (e.g., evoked potentials and nerve conduction) can be utilized to monitor progress. For examples of neuropathological examinations, see, e.g., U.S. Patent Application No. 2007/023711; Schellenberg et al. (2007) Magnetic resonance imaging of blood-spinal cord barrier disruption in mice with experimental autoimmune encephalomyelitis, Magn. Reson. Med. 58:298-305; and Larsen et al. (2003) Matrix metalloproteinase-9 facilitates remyelination in part by processing the inhibitory NG2 proteoglycan, J. Neuroscience 23: 11127-35; Brundula et al., Brain 125:1297, (2002); Giuliani et al., J Neuroimmunol 165:83, (2005).

As a general rule, the dosage ranges for the administration of the compositions are those large enough to produce the desired effect in which the symptoms of the disease are affected. For example, functional improvement, histological improvement, gene expression, and the like can be affected and monitored to determine the desired outcome. The dosage should not be so large as to cause adverse side effects, such as unwanted cross-reactions and unwanted inflammatory reactions. Dosage can vary, dependent on the route of administration used.

In a preferred embodiment of the first aspect of the invention, the (poly)peptide is to be administered at dose of between 100 µg/kg body-weight and 2000 µg/kg body-weight. In context of the present invention, the treatment of the mouse EAE model with EGF was found to be effective at all the tested dosages of 2.5 ug/mouse and 10 ug/mouse, as detailed in Examples 1 and 2. With an average mouse body-weight (bd wt) of about 20 g, these dosages correspond to about 125 ug/kg bd wt and about 500 ug/kg bd wt. It is, hence, expected that dosages in the range as indicated in the above preferred embodiment may generally be therapeutically effective in mammalian subjects, in particular in human subjects.

Further examples of doses of the (poly)peptide include about 50-5000 µg/kg bd wt, about 75-4000 µg/kg bd wt, about 100-3000 µg/kg bd wt, and more particularly about 100-1000 µg/kg bd wt (or any amount in between), or any dose that achieves a blood level in the range of that measured in healthy subjects, i.e. approximately 30 pg/ml. In a recent report, 15 healthy subjects were found to have mean levels of EGF of 32.66 (79.82) pg/ml in serum and of 1371.64 (1205.48) pg/ml in saliva (B 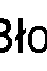 ochowiak KJ, *et al.,* Adv Clin Exp Med. (2018) Apr;27(4):455-461.

Nucleic acids encoding the (poly)peptide of the invention may be administered using gene therapy methods. Retrovirus vectors and adeno-associated virus (AAV) vectors are preferred vectors according to the invention for transferring nucleic acids encoding the (poly)peptides of the invention into cells *in vivo,* particularly into human cells. These vectors provide efficient delivery of genes into cells, and the transferred nucleic acids are stably integrated into the chromosomal DNA of the host. The development of specialized cell lines ("packaging cells") that produce replication-defective retroviruses are especially preferred for gene therapy applications (see, e.g., Miller, A. D. Blood 76:271 (1990)). Recombinant retrovirus may be constructed in which part of the retroviral coding sequence (gag, pol, env) has been replaced by nucleic acid encoding one of the subject receptors rendering the retrovirus replication defective. The replication defective retrovirus is then packaged into virions which can be used to infect a target cell through the use of a helper virus by standard techniques. Protocols for producing recombinant retroviruses and for infecting cells *in vitro* or *in vivo* with such viruses may be found, e.g., in Current Protocols in Molecular Biology, Ausubel, F. M. et al. (eds.) Greene Publishing Associates, (1989), Sections 9.10-9.14 and other standard laboratory manuals. Representative examples of retroviruses include pLJ, pZIP, pWE and pEM which are well known to those skilled in the art. Representative examples of packaging virus lines for preparing both ecotropic and amphotropic retroviral systems include psi.Crip, psi.Cre, psi 2 and psi.Am. Retroviruses have been widely used to introduce a variety of genes into many different cell types *in vitro* and/or *in vivo.* Moreover, it is useful to limit the infection spectrum of retroviruses and retroviral-based vectors by modifying the viral packaging proteins on the surface of the viral particle (see, for example PCT publications WO93/25234 and WO94/06920; Roux et al. PNAS 86:9079-9083 (1989); Julan et al. J. Gen Virol 73:3251-3255 (1992); and Goud et al. Virology 163:251-254 (1983); Neda et al. J. Biol Chem 266:14143-14146 (1991)).
Demyelinating disorders can be classified according to whether the demyelination is due to inflammatory processes. Exemplary inflammatory demyelinating disorders are, without limitation, multiple sclerosis (MS), acute-disseminated encephalomyelitis (ADEM) and acute haemorrhagic leucoencephalitis (AHL).

In accordance with the first aspect of the invention, the demyelinating disorder is an inflammatory demyelinating disorder: the EAE mouse model used herein (see Examples 1 and 2) is characterized by inflammatory demyelination that could be ameliorated by EGF administration. In an alternative embodiment not part of the claimed invention, the demyelinating and/or neurodegenerating disorder is a non-inflammatory disorder.

Demyelinating disorders can also be classified depending on the underlying reason for demyelination in myelinoclastic and leukodystrophic diseases. In demyelinating myelinoclastic diseases, a normal and healthy myelin is destroyed by a toxic, chemical, or autoimmune substance. In demyelinating leukodystrophic diseases (also known as dysmyelinating diseases), myelin is abnormal and degenerates. Exemplary demyelinating myelinoclastic diseases are, without limitation, multiple sclerosis (MS), Schilder's disease (myelinoclastic diffuse sclerosis), Devic's disease, Balo's disease and other and other disorders with immune system involvement. Exemplary demyelinating leukodystropic diseases are, without limitation, CNS neuropathies like those produced by vitamin B12 deficiency, central pontine myelinolysis, tabes dorsalis (syphilitic myelopathy), leukoencephalopathies (such as progressive multifocal leukoencephalopathy) and leukodystrophies.

In accordance with another preferred embodiment of the first aspect of the invention, the inflammatory demyelinating disorder is a myelinoclastic disorder: the MOG-induced EAE mouse model as used herein (Examples 1 and 2) is characterized by myelinoclastic demyelination which could be ameliorated by EGF administration. In other instances, the inflammatory demyelinating disorder is a leukodystrophic disorder.

Demyelinating disorders (or diseases) can also be divided in those affecting the central nervous system (CNS) and those affecting the peripheral nervous system (PNS), presenting different demyelination conditions. The demyelinating diseases of the CNS include, without limitation, myelinoclastic disorders, such as multiple sclerosis (MS), Devic's disease and other disorders with immune system involvement called inflammatory demyelinating diseases, leukodystrophic disorders in which myelin is not properly produced such as CNS neuropathies like those produced by vitamin B12 deficiency, central pontine myelinolysis, myelopathies like tabes dorsalis (syphilitic myelopathy), leukoencephalopathies (such as progressive multifocal leukoencephalopathy) and leukodystrophies. These disorders are often associated with the conditions optic neuritis and transverse myelitis, which are inflammatory conditions, because inflammation and demyelination are frequently associated. Some of them are idiopathic (arising spontaneously, without known cause) and for some others the cause has been found, like some cases of neuromyelitis optica. The demyelinating diseases of the PNS include, without limitation, Guillain-Barré syndrome (GBS) and its chronic counterpart, chronic inflammatory demyelinating polyneuropathy (CIDP), Anti-MAG peripheral neuropathy, Charcot-Marie-Tooth disease (and its counterpart Hereditary neuropathy with liability to pressure palsy), copper deficiency-associated conditions (e.g., peripheral neuropathy, myelopathy, rarely optic neuropathy etc.), and progressive inflammatory neuropathy. Although multiple sclerosis (MS) is usually limited to the CNS, there are rare cases where also the PNS is affected.

In accordance with another preferred embodiment of the first aspect of the invention, the inflammatory demyelinating disorder is a disorder of the central nervous system (CNS): the MOG-induced EAE mouse model as used herein (Examples 1 and 2) is characterized by CNS demyelination and neurodegeneration that could be ameliorated/prevented by the treatment with EGF. It is, however, expected that EGF treatment is also effective in ameliorating/preventing demyelinating and/or neurodegenerating disorders involving the peripheral nervous system (PNS). Hence, in other instances, the inflammatory demyelinating disorder is a disorder of the of the peripheral nervous system (PNS).

In accordance with another preferred embodiment of the first aspect of the invention, the inflammatory demyelinating disorder is an immune disease, preferably an auto-immune disease. In a more preferred embodiment of the first aspect of the invention, the immune disease is (a) multiple sclerosis (MS), (b) Guillain-Barré syndrome (GBS); or (c) chronic inflammatory demyelinating polyneuropathy (CIDP).

MS is a chronic inflammatory demyelinating immune (mostly auto-immune) disease of the CNS (and rarely also affecting the PNS) of unknown etiology and heterogeneous clinical symptoms and course. Depending upon clinical presentation and course, MS is classified either as relapsing remitting (RR), primary progressive (PP), or secondary progressive (SP). Approximately 85% of MS patients initially exhibit a RR course, characterized by acute attacks (relapse) followed by partial or full recovery (remission), which occurs at variable intervals. Of these RR-MS patients, about two-thirds of the untreated patients transition to the secondary progressive phase at which time neurologic disability accumulates in the absence of attacks. About 15% of MS patients have a primary progressive course manifested by progressive clinical worsening from onset with no clinical attacks (Mayo L, et al., Immunol Rev. (2012);248(1):170-87). As used herein, clinically isolated syndrome (CIS) refers to: 1) a single clinical attack suggestive of MS and 2) at least one lesion suggestive of MS. As an example, the patient has at least 1 cerebral lesion detectable by an MRI scan and suggestive of multiple sclerosis. As an additional example the lesion is associated with brain tissue inflammation, myelin sheath damage or axonal damage. As another example the lesion is a demyelinating white matter lesion visible on brain MRI. The term "single clinical attack" is used synonymously with "first clinical episode", "first clinical attack", and "first clinical event" which, for example, presents as a clinical episode of optic neuritis, blurring of vision, diplopia (double vision), involuntary rapid eye movement, blindness, loss of balance, tremors, ataxia, vertigo, clumsiness of a limb, lack of coordination, weakness of one or more extremity, altered muscle tone, muscle stiffness, spasms, tingling, paraesthesia, burning sensations, muscle pains, facial pain, trigeminal neuralgia, stabbing sharp pains, burning tingling pain, slowing of speech, slurring of words, changes in rhythm of speech, dysphagia, fatigue, bladder problems (including urgency, frequency, incomplete emptying and incontinence), bowel problems (including constipation and loss of bowel control), impotence, diminished sexual arousal, loss of sensation, sensitivity to heat, loss of short term memory, loss of concentration, or loss of judgment or reasoning. As used herein, the criteria, as defined by Poser et al. Neurology, (1983), 13 (3): 227-230, used to determine if a subject meets the condition consistent with clinically definite multiple sclerosis (CDMS) are: (i) two attacks and clinical evidence of two separate lesions; or (ii) two attacks; clinical evidence of one lesion and paraclinical evidence of another separate lesion. An attack (also referred to as an exacerbation, flare, or relapse) is defined clinically as the sudden appearance or worsening of a symptom or symptoms of neurological dysfunction, with or without objective confirmation. Patients with CIS that experience a second clinical attack are generally considered to have clinically definite multiple sclerosis (CDMS). Over 80 percent of patients with a CIS and MRI lesion go on to develop MS, while approximately 20 percent have a self-limited process (Brex et al., N Engl J Med. (2002), 346(3):158-64; Frohman, et al., Neurology. (2003), 61(5):602-11).

Guillain-Barré syndrome (GBS) is an immune-mediated disorder of the PNS characterized by neuromuscular weakness and frequently accompanied by flaccid paralysis and may occasionally lead to death. It is classified as an acute inflammatory demyelinating polyneuropathy with a variant form designated as acute motor axonal neuropathy (Hughes and Cornblath, 2005. Lancet 366:1653-1666). Anti-ganglioside antibodies have been proposed as contributors to GBS pathogenesis (Kaida et al., 2009. J. Neuroimmunol. 182:212-218; Ariga and Yu, 2005. J. Neurosci. Res. 80:1-17). Gangliosides are abundantly expressed in human nerves (Yu et al., 2004. J. Lipid Res. 45:783-793) and generally believed to have important roles as mediators of cell adhesion and modulators of signal transduction (Regina Todeschini and Hakomori 2008. Biochim. Biophys. Acta 1780:421-433). The etiology of GBS is complex and not fully known. A growing body of evidence, however, indicates that aberrant immune responses triggered by an infectious agent or vaccination allow disease development and the underlying pathogenetic mechanisms (Langmuir et al., 1984. Am J Epidemiol 119: 841-879; Kuwabara, 2004. Drugs 64: 597-610; Souayah et al., 2007. Vaccine 25: 5253-5255). The most commonly identified microbial agents are Campylobacter jejuni (C. jejuni), Haemophilus influenzae, cytomegalovirus (CMV), Epstein-Barr virus, and Mycoplasma pneumoniae (Hughes et al., 1999. J Neuroimmunol 100: 74-97; Hadden et al., 2001. Neurology 56: 758-765; Sivadon-Tardy et al., 2006. Emerg Infect Dis 12: 990-993; Sivadon-Tardy et al., 2009. Clin Infect Dis 48: 48-56). A preceding infectious event and patient-related host factors also seem to be related to certain subtypes of GBS and may affect the severity of the disease (Geleijns et al., 2005. Neurology 64: 44-49; Caporale et al., 2006. J Neuroimmunol 177: 112-118; Yuki, 2007. Muscle Nerve 35: 691-711). C. jejuni infection frequently induces anti-ganglioside antibodies in the patient's serum (Yuki et al., 1990. Neurology 40: 1900-1902; Usuki et al., 2006. J Neurosci Res 83: 274-284). Thus, despite the possibility of other pathogenic mechanisms, an antibody-mediated process is one of the major insults to the nerve, causing both conduction block and velocity loss and the ensuing clinical symptoms (Rinaldi and Willison, 2008. Curr Opin Neurol 21: 540-546; van Doorn et al., 2008. Lancet Neural 7: 939-950; Kaida et al., 2009. Glycobiology 19: 676-692).

Chronic inflammatory demyelinating polyneuropathy (CIDP) is an acquired immune-mediated inflammatory disorder of the PNS. The disorder is sometimes called chronic relapsing polyneuropathy (CRP) or chronic inflammatory demyelinating polyradiculoneuropathy (because it involves the nerve roots) (Kissel JT, et al., Seminars in Neurology (2003), 23(2): 169-80). CIDP is closely related to Guillain-Barré syndrome and it is considered the chronic counterpart of that acute disease. Several variants have been reported. An asymmetrical variant of CIDP is known as Lewis-Sumner Syndrome. There is also a variant with CNS involvement named combined central and peripheral demyelination (CCPD). In some cases, the CIDP is multifocal acquired demyelinating sensory and motor neuropathy. In some examples, the CIDP is induced by HIV infection.

In accordance with another preferred embodiment of the first aspect of the invention, the MS is of the phenotype (a) Clinically Isolated Syndrome (CIS), (b) Primary Progressive MS (PPMS), (c) Secondary Progressive MS (SPMS), (d) Relapsing-Remitting MS (RRSS), or (e) an intermediate form of any combination of (a) to (d).

In accordance with another preferred embodiment of the first aspect of the invention, the inflammatory demyelinating disorder is associated with a shortage or deficiency of (a) Epidermal Growth Factor (EGF); and/or (b) cobalamin (Cbl) (Vitamin B₁₂). With a "shortage" of EGF or cobalamin, as used herein, is meant a decrease of at least 50%, preferably of at least 75%, of the level of these substances as compared to their level(s) determined in one or more samples (e.g., in body fluid, preferably cerebrospinal fluid) from one or more healthy subject(s). Methods for determining the levels of EGF and/or cobalamin, e.g., radioimmunoassays, are known in the art and described, for example, in Scalabrino G, et al.; Brain Res. (2010);1333:64-71, and commercially available (radioimmunoassay kits for determination of Vitamin B12 levels (Diagnostic Products Corporation, USA) and EGF levels (Penisula Laboratories, San Carlos, CA)).

The invention is herein described, by way of example only, with reference to the accompanying drawings for purposes of illustrative of the preferred embodiments of the present invention.

The Figures show
**Fig. 1****.** Effects of late prophylactic treatment with EGF on body weight (BW) variation in MOG-induced EAE. Statistical differences among groups were assessed using T-test.
**Fig. 2****.** Effects of late prophylactic treatment with EGF on duration of disease in MOG-induced EAE. Statistical differences among groups were assessed using Kruskal-Wallis and Dunn's post-hoc.
**Fig. 3****.** Effects of EGF treatment on onset of the disease in MOG-induced EAE until day 46 post immunization. Statistical differences among groups were assessed using Kruskal-Wallis and Dunn's post-hoc.
**Fig. 4****.** Effects of late prophylactic treatment with EGF on clinical score in MOG-induced EAE [Mean value per day is shown]. Statistical differences among groups were assessed using Kruskal-Wallis and Dunn's post-hoc.
**Fig. 5****.** Effects of late prophylactic treatment with EGF on the cumulative score in MOG-induced EAE. Statistical differences among groups were assessed using Kruskal-Wallis and Dunn's post-hoc.
**Fig. 6****.** Effects of late prophylactic treatment on the histological score (damage score) in a mouse model of MOG-induced EAE.

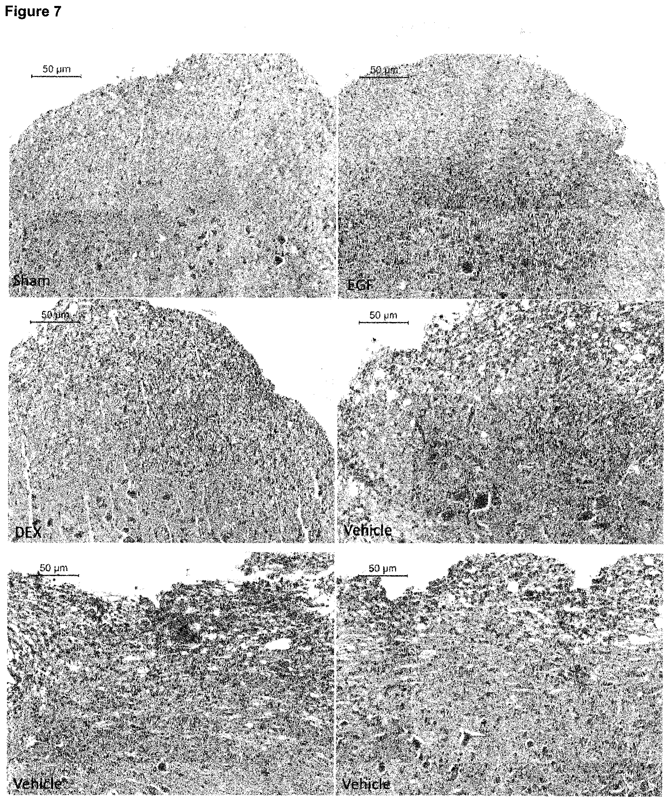

**Fig. 7****.** Representative images of hematoxylin and eosin (H&E) stained sections of spinal cord (SC). Treatment with EGF has improved inflammatory cell infiltration caused by the EAE. The severity of the damage was assessed 50 days after EAE induction by staining with hematoxylin and eosin. Sham mice did not show histological changes in spinal cord tissues. Significant damage was observed in EAE mice, as demonstrated by the presence of numerous inflammatory foci in the white matter of the spinal cord. In particular, a complete reduction of inflammatory cell infiltration, such as T lymphocytes and monocytes, was observed in samples taken from EAE mice treated with the most effective (or "golden") dose of EGF of 2.5ug/mouse EOD. Magnification: 10×; scale bar: 50 µm. Values are expressed as means ± SEM and calculated in 20 spinal cord sections per mouse.
**Fig. 8****.** Effects of EGF late prophylactic treatment on the demyelination score in MOG-induced EAE.
**Fig. 9****.** Representative images of Luxol fast blue (LFB) stained sections of spinal cord (SC). EGF attenuated the degradation of myelin caused by the EAE. The sections of the SC collected at the end of the treatment on the day of sacrifice were analyzed with LFB staining to detect demyelination. In sham and DEX control groups, they do not show demyelination plaques. The staining with LFB shows the formation of demyelination plaques occurred in the EAE group. Myelin degradation was completely attenuated in EAE mice treated with EGF. Magnification: 10×; scale bar: 50 µm

The examples illustrate the invention:

### Example 1: Materials and Methods

### Animal Study

### Acclimatization, housing and feeding

Six to 7 weeks old female C57BL/6 mice weighing between 16 to 18 grams and purchased from Envigo (San Pietro al Natisone, Udine, Italy) were kept at the animal facility of the Department of Biomedical and Biotechnological Sciences, University of Catania, under standard laboratory conditions (non-specific pathogen free) with free access to food and water and were allowed to adapt for at least one week to their environment before commencing the study.

Animals were housed within a limited-access rodent facility under controlled microbial conditions, which excluded murine pathogens, and will be kept in groups of maximum 5 mice, in polycarbonate cages (Tecniplast, Varese, Italy), according to the Italian legislation (each mouse is to have a surface of 180 cm² with minimum 12 cm height). This regimen does not exclude various species of Helicobacter. Cages are sterilized and filled with wood shavings as bedding material. Automatically controlled environmental conditions are set to maintain temperature at 20 - 24°C with a relative humidity (RH) of 30 - 70 %, 10-30 air changes /hr and a natural dark:light cycle.

Protection of animals used in the experiment is in accordance with Directive 2010/63/UE, enforced by the Italian D. Lgs 26/2014. Physical facilities and equipment for accommodation and care of animals are in accordance with the provisions of EEC Council Directive 86/609.

### Induction of MOG-induced EAE

Mice were immunized by s.c. injection of an emulsion composed of 200 µg MOG₃₅₋₅₅ peptide (Genemed Synthesis, San Francisco, CA) in Complete Freund's Adjuvant (CFA, Difco, Detroit, U.S.A.) containing 0.5 mg (per injection) of *Mycobacterium tuberculosis.* Each mouse received subcutaneous injections of 200 ul emulsion divided among two sites, left and right flank, (50%/50%) draining into the axillary lymph nodes. Pertussis toxin (Calbiochem, Nottingham, UK) was used as a co-adjuvant and was administered i.p. at the dose of 200 ng/mouse on day 0 and 200 ng/mouse on day 2 post immunization (Donia M, Mangano K, Quattrocchi C, Fagone P, Signorelli S, Magro G, Sfacteria A, Bendtzen K, Nicoletti F. Specific and strain-independent effects of dexamethasone in the prevention and treatment of experimental autoimmune encephalomyelitis in rodents. Scand J Immunol. 2010 Nov;72(5):396-407*).*

### Study design

Six groups of 10-12 animals each (missing mice died because of anaesthesia or pertussis toxin toxicity) were treated under a late prophylactic regime from 7 days after immunization to day 50, as described in Table 1 below:

**Table 1:**

| **Group** | **Number** | **Treatment** | **Dose** | **Route** | **Administration frequency** |
|---|---|---|---|---|---|
| **1** | 12 | **EGF** | **2.5 µg/mouse** | **i.p.** | Every other day |
| **2** | 10 | **EGF** | **10 µg/mouse** | **i.p.** | Every other day |
| **3** | 11 | **EGF** | **2.5 µg/mouse** | **i.p.** | Daily, 5 times a week |
| **4** | 11 | **EGF** | **10 µg/mouse** | **i.p.** | Daily, 5 times a week |
| **5** | 12 | **Dex** | **0.6 mg/kg** | **i.p.** | Daily, 5 times a week |
| **6** | 12 | **Vehicle** | **----** | **i.p.** | Daily, 5 times a week |
| **7** | 5 | **SHAM** | | | |

Five additional mice were considered as SHAM. These mice, on the day of EAE induction, were anesthetized as other mice, but were injected only with Freund adjuvant without *Mycobacterium tuberculosis* and without MOG. These untreated mice were considered as healthy mice. The number of animals per group is the minimum allowing an accurate assessment of observed pharmacological effects.

Dexamethasone (Soldesan, Dex) was purchased from a local pharmacy and was used as positive control drug as previously described (Donia M, Mangano K, Quattrocchi C, Fagone P, Signorelli S, Magro G, Sfacteria A, Bendtzen K, Nicoletti F. Specific and strain-independent effects of dexamethasone in the prevention and treatment of experimental autoimmune encephalomyelitis in rodents. Scand J Immunol. 2010 Nov;72(5):396-407)*.*

*Recombinant human EGF with EC₅₀: 0.08-0.8 ng*/*ml as assessed on the growth on mouse fibroblast cell lines BALB*/*3T3*) (Sigma Aldrich, Milan, Italy) was dissolved in water for injection and was administered by i.p. injection as described in Table 1 above.

Previous provided experiments evidenced that EGF is active on rat CNS even when it is administered by i.p. route, thus entailing its crossing the BB barrier (Scalabrino G., Lorenzini E.C., Monzio-Compagnoni B., Colombi R.P., Chiodini E., Buccellato F.R. Subacute combined degeneration in the spinal cord of totally gastrectomized rats. Ornithine decarboxylase induction, cobalamin status, and astroglial reaction. Lab. Invest. 72, (1995), pp.114-123)

### Endpoints

### Clinical Assessment

Starting from day 7 post-immunization, the animals were examined individually for the presence of paralysis according to the following score:
0 = no sign of disease; 0.5 = partial tail paralysis ; 1 = tail paralysis; 1.5 = tail paralysis + partial unilateral hind limb paralysis; 2 = tail paralysis + hindlimb weakness or partial hindlimb paralysis; 2.5 = tail paralysis + partial hindlimb paralysis (lowered pelvis); 3 = tail paralysis + complete hindlimb paralysis; 3.5 = tail paralysis + complete hindlimb paralysis + incontinence; 4 = tail paralysis + hindlimb paralysis + weakness or partial paralysis of forelimbs; 5 = moribund or dead (Donia M, Mangano K, Quattrocchi C, Fagone P, Signorelli S, Magro G, Sfacteria A, Bendtzen K, Nicoletti F. Specific and strain-independent effects of dexamethasone in the prevention and treatment of experimental autoimmune encephalomyelitis in rodents. Scand J Immunol. 2010 Nov;72(5):396-407*).* Clinical signs were monitored daily and body-weight was monitored three times a week. Mice reaching a score of 4 and/or losing more than 20% of their body-weight were monitored daily by the veterinary and subcutaneously treated with saline and, if necessary, ethically euthanized. The clinical readouts for this study include the clinical score, cumulative score, incidence, duration and onset of the disease and variation of the body weight throughout the study period.

### Histological analysis

Mice were sacrificed and brain and the spinal cords were resected and stored in 4% formaldehyde (Sigma, Milano, Italy) at 4 °C for 24 h. To assess inflammation, serial 5-µm cross sections were stained with hematoxylin and eosin (H&E) (Mangano K, Fagone P, Bendtzen K, Meroni PL, Quattrocchi C, Mammana S, Di Rosa M, Malaguarnera L, Coco M, Magro G, Di Marco R, Nicoletti F. Hypomethylating agent 5-aza-2'-deoxycytidine (DAC) ameliorates multiple sclerosis in mouse models. J Cell Physiol. 2014 Dec;229(12):1918-25*).* Semiquantitative histological evaluation was performed using the following scores based on the severity of inflammation: 0, no inflammation; 1, few cells; 2, moderate, perivascular cuffing 3, dense inflammatory cellular infiltrates, parenchymal necrosis *(*Mangano K, Fagone P, Bendtzen K, Meroni PL, Quattrocchi C, Mammana S, Di Rosa M, Malaguarnera L, Coco M, Magro G, Di Marco R, Nicoletti F. Hypomethylating agent 5-aza-2'-deoxycytidine (DAC) ameliorates multiple sclerosis in mouse models. J Cell Physiol. 2014 Dec;229(12):1918-25*).* To assess the severity and extent of demyelination, myelin structure was observed by Luxol fast blue (Lfb) staining (Bio-Optica) and scored using a semiquantitative grading system: 0=normal; 1=demyelinated fibres less than 25%; 2=demyelinated fibres 25-50%; 3=demyelinated fibres 50-75%; 4=demyelinated fibres more than 75% (Furlan R, Poliani PL, Galbiati F, Bergami A, Grimaldi LM, Comi G, Adorini L, Martino G. Central nervous system delivery of interleukin 4 by a non replicative herpes simplex type 1 viral vector ameliorates autoimmune demyelination. Hum Gene Ther. 1998 Nov 20;9 (1):2605-17)*.* Cross sections of spinal cord were analyzed using a light microscope (LEICA DM 2000 combined with LEICA ICC50 HD camera). Images (n = 20 images from each group) were acquired for the evaluation of the percentage of demyelination using the Leica Application Suite V4.2.0 software. Histological analyses were only performed in sham-treated mice, and the groups of mice treated with vehicle, EGF eod (every-other-day) at 2.5 ug/mouse and Dex.

### Statistical analysis

The results were presented as mean ± SD. During the study, an initial statistical evaluation was carried out by Student's T-test. At the end of the study, a more selective statistical analysis was carried out. Statistical differences in the incidence of disease among groups were assessed using the Fisher Exact test for each group comparison. Data have been subjected to a normality test for the assessment of their Gaussian distribution. The Shapiro Wilk, Kolmogorov Smirnov and D' Agostino Pearson omnibus tests were used. Based on the distribution of the data, either the T-test test (for parametric data) or the Kruskal-Wallis test, followed by Dunn's multiple comparison test (for non-parametric data) were performed. A p value <0.05 has been considered for statistical significance. All analyses have been performed using the Prism GraphPad v5 software or Microsoft Excel.

### Example 2: Animal Study

Although, at the beginning of the treatment, no clinical signs of EAE were observed, both the literature data from the present inventors and others (Mangano K, et al. J Cell Physiol. (2014);229(12):1918-25; Donia M, et al., Scand J Immunol. (2010);72(5):396-407; Eugster HP, et al., Eur J Immunol. (1999);29(2):626-32), and the initial observation of EAE occurrence in control mice already from the 9^{th} day post-immunization, indicated that immunoinflammatory encephalytogenic events were fully ongoing at the time the treatment with EGF was started.

The EAE model worked well, although the clinical course was more aggressive compared to previous studies (Mangano K, et al., J Cell Physiol. (2014);229(12):1918-25*;* Donia M, et al., Scand J Immunol. (2010);72(5):396-407*)* and this must be taken into consideration when examining the effects of EGF on the course of the disease.

The treatment with EGF administered every-other-day (EOD) from day 7 post-immunization at the dose of 10 ug/mouse, and mostly at the lower dose of 2.5 ug/mouse, induced an amelioration of the clinical course of the disease, as detailed below. The mice treated with Dex also exhibited a strong amelioration of clinical readouts. Sham mice, injected only with Freund adjuvant without MOG, as expected, did not develop disease.

### Toxicity

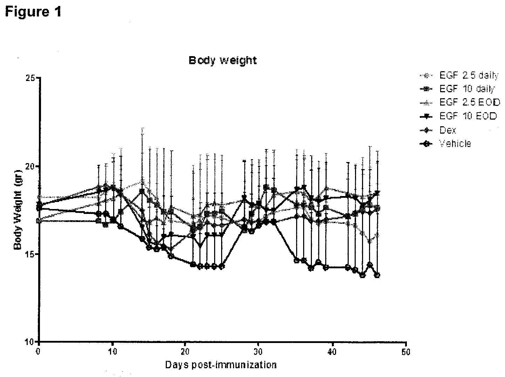

The i.p. treatment with test compounds appeared to be well tolerated throughout the entire study, as judged by the clinical status of the mice and by body-weight (BW) variation (Figure 1).

### Disease Incidence

Disease incidence was determined by summing up the number of animals per group that ever showed a score ≥ 0.5 throughout the whole experiment. The determined incidences showed that the MOG-EAE model worked well. No significant effects were observed in the incidence of the disease in groups of mice treated with both doses and both regimens of EGF and with the positive control drug Dexamethasone (Dex), as compared to vehicle-treated mice.

### Disease Duration

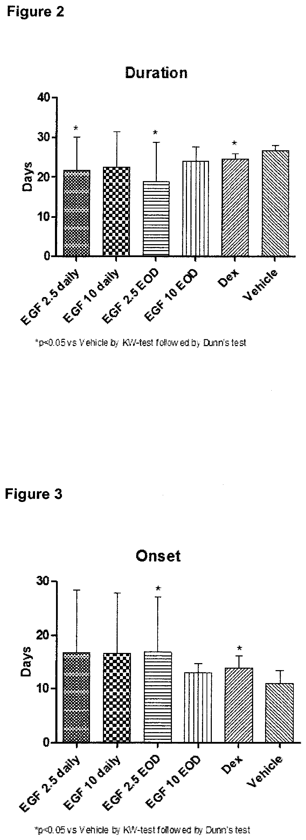

The duration of the disease was calculated for each animal by counting the number of days on which the animal showed a clinical score higher than 0. Finally, the mean for each treatment group was calculated. The treatment with EGF at the dose of 2.5 ug/mouse both administered daily and every-other-day (EOD), significantly reduced the duration of the disease. The treatment with the positive control drug Dexamethasone (Dex) also showed a statistically significant reduction of the disease duration as compared to vehicle-treated control mice (see Figure 2).

### Disease Onset

Disease onset was determined as the first day on which an animal showed a clinical score ≥ 0.5. For animals that never showed symptoms, onset was considered to be the last day of observation. The onset of the disease was significantly delayed in the group of mice treated with EGF 2.5 ug/mouse EOD and with the positive control drug Dexamethasone (Dex) as compared to vehicle-treated mice (see Figure 3).

### Clinical Score

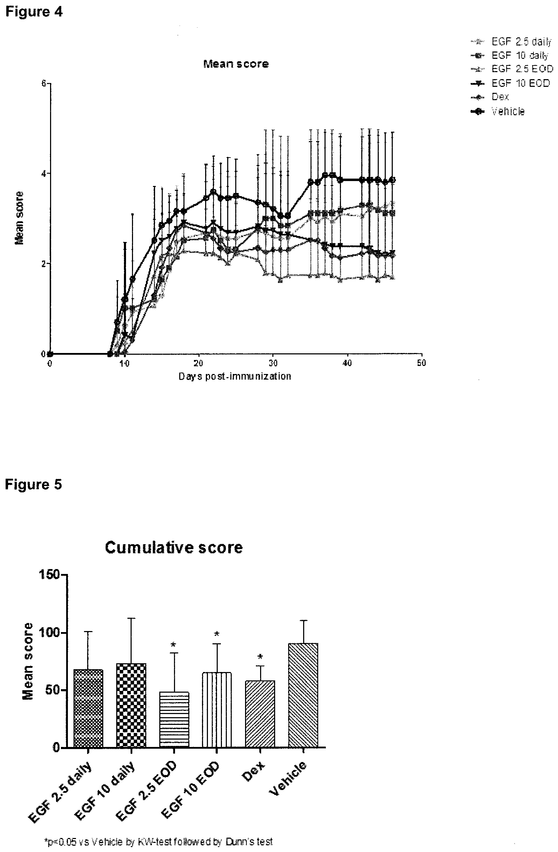

Classical signs of EAE started to appear in the vehicle-treated control animals 9 days after the immunization (see Figure 4). The daily treatment with EGF at the dose of 2.5 ug, significantly reduced the clinical score only at the begin of the disease between day 14 to 16 and, at the dose of 10 ug, from day 14 to 16 and at 24 and 25 days post immunization. Stronger effects were observed when EGF administered every other day, in particular, the dose of 2.5 ug significantly reduced the clinical score from day 9 to 11 and from day 21 until the end of the study and the higher dose of 10 ug significantly reduced the clinical score from day 9 to 11 and from day 34 until the end of the study (Figure 4 and Table 2). Dex exhibited a significant lower clinical score through the entire treatment period by Kruskal-Wallis analysis (Figure 4 and Table 2) compared to vehicle-treated mice.

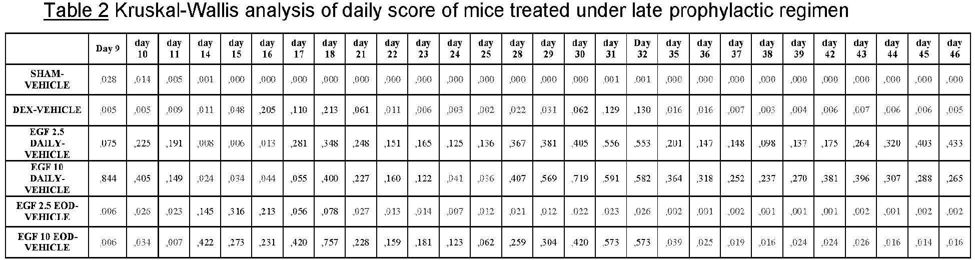

### Cumulative Score

The cumulative clinical score was calculated for each mouse by adding the daily scores from the day of onset (score of disease ≥ 0.5) until the end of treatment. The late prophylactic treatment with EGF at the dose of 2.5 and 10 ug/mouse administered every other day as well as with Dex showed a reduction of the cumulative score compared to vehicle, which was statistically significant via the Kruskal-Wallis test (Figure 5). No significant effects were observed upon the daily treatment with EGF at both doses.

### Histology

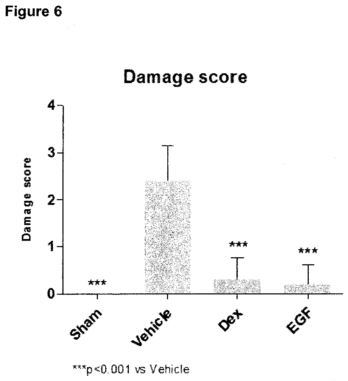

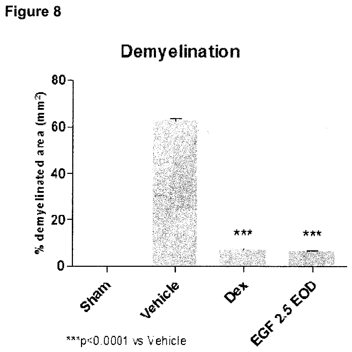

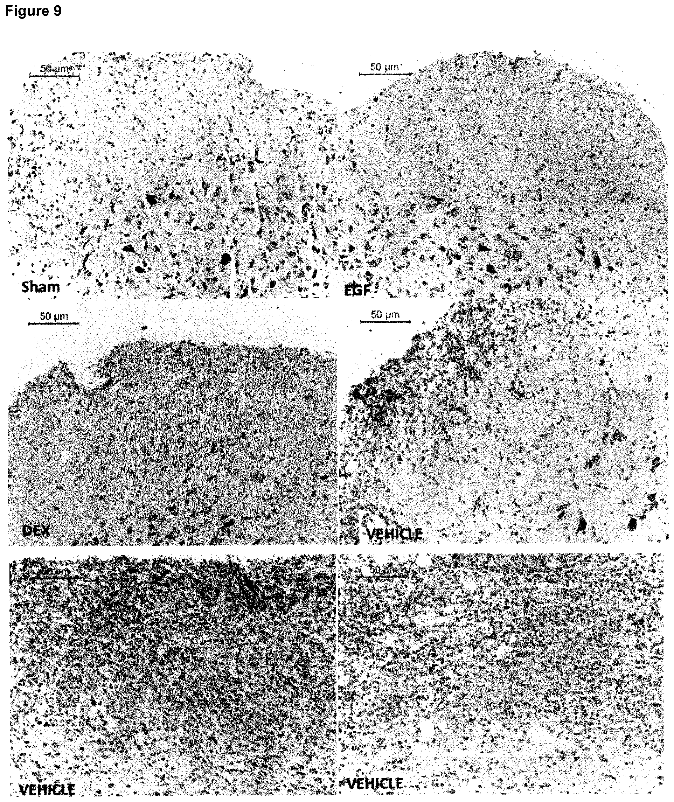

The inflammation and the demyelination score for each mouse is the rounded mean of the score of three sections of the respective mouse. No cell infiltrate was found in the brains of mice from all experimental groups. The H&E sections of the spinal cord showed that the mice treated with the vehicle underwent infiltration of lymphocytes into the white matter of SC. In contrast, the histological scores of damage were significantly reduced in EGF 2.5 ug/mouse in comparison to vehicle-treated mice. The treatment with EGF induced a significant reduction in the infiltration of leukocytes SC similar to the positive control drug Dexamethasone (Dex). EGF-treated mice, in comparison to Dex-treated group, also exhibit a greater reduction of the edema of the white matter score (Figures 6 and 7). Myelin structure was observed by Luxol fast blue (Lfb) staining (Bio-Optica) and followed by scoring to analyze the degree of demyelination. Severe demyelination was detected in the group treated with the vehicle (Figures 8 and 9). The administration of EGF and Dex significantly reduced the degree of demyelination as compared to the group treated with the vehicle.

Citation of the above patents, patent applications, publications and documents is not an admission that any of the foregoing is pertinent prior art, nor does it constitute any admission as to the contents or date of these publications or documents. Their citation is not an indication of a search for relevant disclosures. All statements regarding the date(s) or contents of the documents is based on available information and is not an admission as to their accuracy or correctness.

The term "a" or "an" can refer to one of or a plurality of the elements it modifies (e.g., "a carrier" can mean one or more carriers) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%).

Certain embodiments of the technology are set forth in the claim(s) that follow(s).

## Claims

1. A (poly)peptide having Epidermal Growth Factor (EGF)-activity or a nucleic acid encoding said (poly)peptide for use in treating or preventing an inflammatory demyelinating disorder, wherein the (poly)peptide
(a) comprises or consists of the amino acid sequence of SEQ ID NO: 1; and/or
(b) comprises or consists of an amino acid sequence, which is at least 66% identical to the amino acid sequence of SEQ ID NO: 1; and/or
(c) comprises or consists of a fragment of (a) or (b); and
wherein the (poly)peptide or nucleic acid is the only pharmaceutically active agent to be administered.

2. The (poly)peptide or nucleic acid for use of claim 1, wherein the fragment of (a) comprises or consists of SEQ ID NO: 2.

3. The (poly)peptide or nucleic acid for use of claim 1 or 2, wherein an effective amount of the (poly)peptide is to be administered to a subject in need thereof once every 36 hours to once every 60 hours.

4. The (poly)peptide for use of claim 1 or 2, wherein the (poly)peptide is to be administered once every 44 hours to once every 52 hours.

5. The (poly)peptide for use of claim 1 or 2, wherein the (poly)peptide is to be administered once every about 48 hours.

6. The (poly)peptide for use of any of claims 1 to 5, wherein the (poly)peptide is to be administered for a period of at least 20 days, preferably at least 30 days, more preferably at least 40 days.

7. The (poly)peptide for use of any of claims 1 to 6, wherein the (poly)peptide is to be administered at dose of between 100 µg/kg body-weight and 2000 µg/kg body-weight.

8. The (poly)peptide for use of any of claims 1 to 7, wherein the inflammatory demyelinating disorder is a myelinoclastic disorder.

9. The (poly)peptide for use of any of claims 1 to 8, wherein the inflammatory demyelinating disorder is a disorder of the central nervous system (CNS).

10. The (poly)peptide for use of any of claims 1 to 9, wherein the inflammatory demyelinating disorder is an immune disease, preferably an auto-immune disease.

11. The (poly)peptide for use of claim 10, wherein the immune disease is
(a) multiple sclerosis (MS);
(b) Guillain-Barré syndrome (GBS); or
(c) chronic inflammatory demyelinating polyneuropathy (CIDP).

12. The (poly)peptide for use of claim 11, wherein the MS is of the phenotype
(a) Clinically Isolated Syndrome (CIS);
(b) Primary Progressive MS (PPMS);
(c) Secondary Progressive MS (SPMS);
(d) Relapsing-Remitting MS (RRSS); or
(e) an intermediate form of any combination of (a) to (d).

13. The (poly)peptide for use of any of claims 1 to 7 and 9, wherein the inflammatory demyelinating disorder is associated with a shortage or deficiency of
(a) Epidermal Growth Factor (EGF); and/or
(b) cobalamin (Vitamin B12).

## Patentansprüche

1. (Poly)peptid mit epidermaler Wachstumsfaktor (*"epidermal growth factor"* (EGF))-Aktivität oder Nukleinsäure, die das (Poly)peptid kodiert, zur Verwendung bei der Behandlung oder Vorbeugung einer inflammatorischen demyelinisierenden Erkrankung, wobei das (Poly)peptid
(a) die Aminosäuresequenz von SEQ ID Nr: 1 umfasst oder aus dieser besteht; und/oder
(b) eine Aminosäuresequenz umfasst oder aus einer Aminosäuresequenz besteht, die zu mindestens 66% mit der Aminosäuresequenz von SEQ ID Nr: 1 identisch ist; und/oder
(c) ein Fragment von (a) oder (b) umfasst oder daraus besteht; und
wobei das (Poly)peptid oder die Nukleinsäure der einzige zu verabreichende pharmazeutische Wirkstoff ist.

2. (Poly)peptid oder Nukleinsäure zur Verwendung nach Anspruch 1, wobei das Fragment von (a) SEQ ID Nr: 2 umfasst oder daraus besteht.

3. (Poly)peptid oder Nukleinsäure zur Verwendung nach Anspruch 1 oder 2, wobei eine wirksame Menge des (Poly)peptids einem Individuum, das dessen bedarf, einmal alle 36 Stunden bis einmal alle 60 Stunden zu verabreichen ist.

4. (Poly)peptid zur Verwendung nach Anspruch 1 oder 2, wobei das (Poly)peptid einmal alle 44 Stunden bis einmal alle 52 Stunden zu verabreichen ist.

5. (Poly)peptid zur Verwendung nach Anspruch 1 oder 2, wobei das (Poly)peptid einmal alle etwa 48 Stunden zu verabreichen ist.

6. (Poly)peptid zur Verwendung nach einem der Ansprüche 1 bis 5, wobei das (Poly)peptid über einen Zeitraum von mindestens 20 Tagen, vorzugsweise mindestens 30 Tagen, stärker bevorzugt mindestens 40 Tagen, zu verabreichen ist.

7. (Poly)peptid zur Verwendung nach einem der Ansprüche 1 bis 6, wobei das (Poly)peptid in einer Dosis zwischen 100 µg/kg Körpergewicht und 2000 µg/kg Körpergewicht zu verabreichen ist.

8. (Poly)peptid zur Verwendung nach einem der Ansprüche 1 bis 7, wobei die inflammatorische demyelinisierende Erkrankung eine myelinoklastische Störung ist.

9. (Poly)peptid zur Verwendung nach einem der Ansprüche 1 bis 8, wobei die inflammatorische demyelinisierende Erkrankung eine Erkrankung des zentralen Nervensystems (ZNS) ist.

10. (Poly)peptid zur Verwendung nach einem der Ansprüche 1 bis 9, wobei die inflammatorische demyelinisierende Erkrankung eine Immunkrankheit, vorzugsweise eine Autoimmunkrankheit, ist.

11. (Poly)peptid zur Verwendung nach Anspruch 10, wobei die Immunkrankheit
(a) Multiple Sklerose (MS);
(b) Guillain-Barré-Syndrom (GBS); oder
(c) chronisch inflammatorische demyelinisierende Polyneuropathie (CIDP) ist.

12. (Poly)peptid zur Verwendung nach Anspruch 11, wobei die MS vom Phänotyp
(a) Klinisch isoliertes Syndrom (KIS);
(b) Primär progrediente MS (PPMS);
(c) Sekundär progrediente MS (SPMS);
(d) Schubförmig-remittierende MS (RRSS); oder
(e) eine Zwischenform einer beliebigen Kombination von (a) bis (d) ist.

13. (Poly)peptid zur Verwendung nach einem der Ansprüche 1 bis 7 und 9, wobei die inflammatorische demyelinisierende Erkrankung mit einem Mangel oder einer Defizienz von
(a) epidermalem Wachstumsfaktor (EGF); und/oder
(b) Cobalamin (Vitamin B12)
verbunden ist.

## Revendications

1. (Poly)peptide ayant une activité de facteur de croissance épidermique (EGF) ou acide nucléique codant ledit (poly)peptide pour une utilisation dans le traitement ou la prévention d'un trouble de démyélinisation inflammatoire, dans lequel le (poly)peptide
(a) comprend ou consiste en la séquence d'acides aminés de la SEQ ID NO : 1 ; et/ou
(b) comprend ou consiste en une séquence d'acides aminés qui est identique à au moins 66 % à la séquence d'acides aminés de la SEQ ID NO : 1 ; et/ou
(c) comprend ou consiste en un fragment de (a) ou (b) ; et
dans lequel le (poly)peptide ou l'acide nucléique est le seul agent actif sur le plan pharmaceutique devant être administré.

2. (Poly)peptide ou acide nucléique pour une utilisation selon la revendication 1, dans lequel le fragment de (a) comprend ou consiste en la SEQ ID NO : 2.

3. (Poly)peptide ou acide nucléique pour une utilisation selon la revendication 1 ou 2, dans lequel une quantité efficace du (poly)peptide est destinée à être administrée à un sujet en ayant besoin une fois toutes les 36 heures ou une fois toutes les 60 heures.

4. (Poly)peptide pour une utilisation selon la revendication 1 ou 2, lequel (poly)peptide est destiné à être administré une fois toutes les 44 heures ou une fois toutes les 52 heures.

5. (Poly)peptide pour une utilisation selon la revendication 1 ou 2, lequel (poly)peptide est destiné à être administré une fois toutes les 48 heures.

6. (Poly)peptide pour une utilisation selon l'une quelconque des revendications 1 à 5, lequel (poly)peptide est destiné à être administré pendant une période d'au moins 20 jours, de préférence d'au moins 30 jours, mieux encore d'au moins 40 jours.

7. (Poly)peptide pour une utilisation selon l'une quelconque des revendications 1 à 6, lequel (poly)peptide est destiné à être administré à une dose comprise entre 100 µg/kg de poids corporel et 2000 µg/kg de poids corporel.

8. (Poly)peptide pour une utilisation selon l'une quelconque des revendications 1 à 7, dans lequel le trouble de démyélinisation inflammatoire est un trouble myélinoclastique.

9. (Poly)peptide pour une utilisation selon l'une quelconque des revendications 1 à 8, dans lequel le trouble de démyélinisation inflammatoire est un trouble du système nerveux central (SNC).

10. (Poly)peptide pour une utilisation selon l'une quelconque des revendications 1 à 9, dans lequel le trouble de démyélinisation inflammatoire est une maladie immune, de préférence une maladie auto-immune.

11. (Poly)peptide pour une utilisation selon la revendication 10, dans lequel la maladie immune est
(a) une sclérose en plaques (SEP) ;
(b) un syndrome de Guillain-Barré (SGB) ; ou
(c) une polyneuropathie démyélinisante inflammatoire chronique (PDIC).

12. (Poly)peptide pour une utilisation selon la revendication 11, dans lequel la SEP est du phénotype
(a) syndrome isolé clinique (SIC) ;
(b) SEP progressive primaire (SEPP) ;
(c) SEP progressive secondaire (SEPS) ;
(d) SEP cyclique (SEPC) ; ou
(e) une forme intermédiaire de n'importe quelle combinaison de (a) à (d).

13. (Poly)peptide pour une utilisation selon l'une quelconque des revendications 1 à 7 et 9, dans lequel le trouble de démyélinisation inflammatoire est associé à un déficit ou une insuffisance en
(a) facteur de croissance épidermique (EGF) ; et/ou
(b) cobalamine (vitamine B12).
